# EUROPEAN PATENT SPECIFICATION

(11) **EP 1 557 145 B1**
(45) Date of publication and mention of the grant of the patent: **18.05.2011**
(21) Application number: 05000791.3
(22) Date of filing: 17.01.2005
(51) Int. Cl.: A61F 5/443, A61F 5/445

(54) **Pouch for medical use**
Beutel für medizinischen Gebrauch
Poche à usage médical

(30) Priority: 21.01.2004 US 537829 P
(43) Date of publication of application: 27.07.2005
(60) Divisional application: 10188121.7
(73) Proprietor: ConvaTec Technologies Inc., Las Vegas, NV 89169 (US)
(72) Inventor: Tsai, Lawrence, Holmdel New Jersey 07733 (US)
(74) Representative: Mays, Julie

(56) References cited:
- EP-A- 0 231 508
- EP-A- 0 272 816
- EP-A- 0 413 250
- DE-A1- 4 400 025
- GB-A- 1 587 604
- GB-A- 2 227 668
- GB-A- 2 301 777
- GB-A- 2 313 061
- US-A- 5 865 819
- US-A- 5 961 502

## Description

### FIELD OF THE INVENTION

The present invention relates to an ostomy pouch for collecting body fluids and/or human waste. One non-limiting aspect of the invention may relate to facilitating disposal of the pouch in a water closet.

### BACKGROUND TO THE INVENTION

The disposing of used medical pouches, such as ostomy pouches that are full of collected human waste, remains an area of concern for pouch users. The disposal should be hygienic yet also convenient for the user. Various attempts have been made to design pouches that may be disposed of by flushing down a water closet, as an easy means of disposal. However, such "flushability" depends on many factors that are generally incompatible with modern pouch requirements. As modern pouches have advanced in order to satisfy customers' needs in terms of security, odour barrier, comfort and aesthetics, the pouches have become more complex, making it more difficult to achieve the desired flushability.

For example, a conventional medical grade adhesive body attachment wafer for attaching the pouch to the body may be made of hydrocolloid polymers for high skin adhesive performance. The wafer may be relatively stiffly conformable, and typically has a thickness of between 1.27 and 2.5 mm (50-100 mils). However, hydrocolloid polymers are not water soluble, and so will remain generally intact in the toilet water. Also, the thickness and characteristics of the wafer mean that it is not easily flushable. Even if the pouch with the wafer is successfully flushed away, the wafer may easily cause the pouch to become trapped in the water closet sewer pipe, risking a blockage. Therefore, pouches using a thick, relatively inflexible hydrocolloid wafer are incompatible with the requirements for pouch flushability.

Even in a two-piece pouch arrangement in which the body attachment wafer is designed to be releasably fastenable to the pouch, some form of connection device is required on the pouch itself to provide the releasable fastening between the pouch and the body attachment wafer. The connection device usually has a certain thickness (typically at least 2 mm, and often much greater), and may include a lateral flange. For optimum security, the connection device should be permanently secured to the pouch. Such a connection device still results in reduced flushability of the pouch, and increases the risk of blockage of the water closet.

Document US 5865819 discloses an ostomy pouch of the state of the art.

Reference is also made to the following patents and applications: US Patent No. 5976118; and US application serial no. 10/630,575. The teachings of these documents may be used in combination with the present invention.

### SUMMARY OF THE INTENTION

The present invention is defined in the claims.

As illustrated in the preferred embodiments, the pouch includes a tear strip or tear tab that is configured to create a tear in the pouch when the tear tab is pulled from a rest position. The tear may allow the escape of air that might otherwise be trapped in the pouch, in order to reduce the buoyancy and volume of the pouch. The tear may also allow the escape of at least some of the pouch contents directly into the toilet water, reducing the volume of the pouch.

The tear tab is configured such that displacement of the tear tab creates an initial single tear. Further displacement of the tear tab causes the tear to propagate in two different directions from the initial tear. This may, for example, enable a relatively wide aperture to be opened from the initial single tear. Such a configuration can generate a wide aperture without the complexity of having to generate multiple tears at different start locations.

The tear tab is joined to a wall of the pouch by one or more lines of attachment. The lines of attachment include a tear initiation line, which may be first line that is torn upon pulling the tear tab. Extending from the tear initiation line are two tear propagation lines, extending in two different propagation directions.

The lines of attachment may, for example, be weld lines. The tear initiation line may have a relatively narrow width, for example, between 1 and 5 mm.

The tear tab may be configured such that, in use, the force required to be applied to the tear tab to create the tearing may be generally in the range of 0.79-7.87 N/cm (2 - 20 N/in) at a tearing speed of 2540 mm/min based on ASTM F88.

The tear tab may comprise a grip portion for enabling the user to grip the tear tab. The grip portion may initially be attached in a stowed condition by a breakable connection. The breakable connection may enable the grip portion to be deployed, e.g. released from the stowed condition, without tearing the pouch envelope, by breaking the breakable connection.

The region of the pouch to be torn open may be configured according to the requirements of the pouch. For example, in one form, the region to be torn open may be positioned near or at a lower portion of the pouch (when the pouch is in a normal upright orientation). Positioning the region to be torn open in this way may allow quick and/or efficient discharge of the pouch contents, which normally drop to the bottom of the pouch envelope. The pouch contents may exude under their weight and/or internal pressure of the pouch. Additionally or alternatively, the region to be torn open may be positioned near or at an upper portion of the pouch (when the pouch is in a normal upright orientation). Positioning the region to be torn open in this way may allow for venting of air from the pouch (e.g. to reduce the pouch's buoyancy in toilet water) while at the same time reducing the risk of the user contacting the pouch contents. The optimum position may depend on the application for which the pouch is intended and/or preferences of users.

The tear tab is joined to the envelope in one or more regions of attachment.

In another form, the pouch with or without the tear strip may be placed in a carrier sleeve or bag before flushing in a toilet. The sleeve or bag may form a slippery layer when exposed to water, thereby sliding on surfaces that might otherwise cause snagging of the pouch.

The above features may be used independently of each other, or they may be combined (as in the preferred embodiments) to yield additional advantages in combination.

### BRIEF DESCRIPTION OF THE DRAWINGS

Non-limiting preferred embodiments of the invention are now described, by way of example only, with reference to the accompanying drawings, in which:
Fig. 1 is a schematic cross-section view of a first embodiment of a pouch;
Fig. 2 is a schematic rear view of the pouch of Fig. 1;
Fig. 3 is a schematic front view of the pouch of Fig. 1;
Fig. 4 is a schematic front view similar to Fig. 3, but showing a tear tab of the pouch in a partly deployed condition;
Fig. 5 is a schematic front view similar to Fig. 4, but showing the tear tab in a fully deployed condition; and
Fig. 6 is a schematic cross-section view of a second embodiment of a pouch;
Fig. 7 is a schematic front view of the pouch components of Fig. 6, showing the outer pouch in an open condition;
Fig. 8 is a schematic front view of a modified example of pouch having a different tear tab configuration;
Fig. 9 is a schematic front view of a pouch having a different tear tab configuration which is not part of the invention; and
Fig. 10 is a schematic front view of a further example of pouch having a different tear tab configuration which is not part of the invention.

### DESCRIPTION OF THE PREFERRED EMBODIMENTS

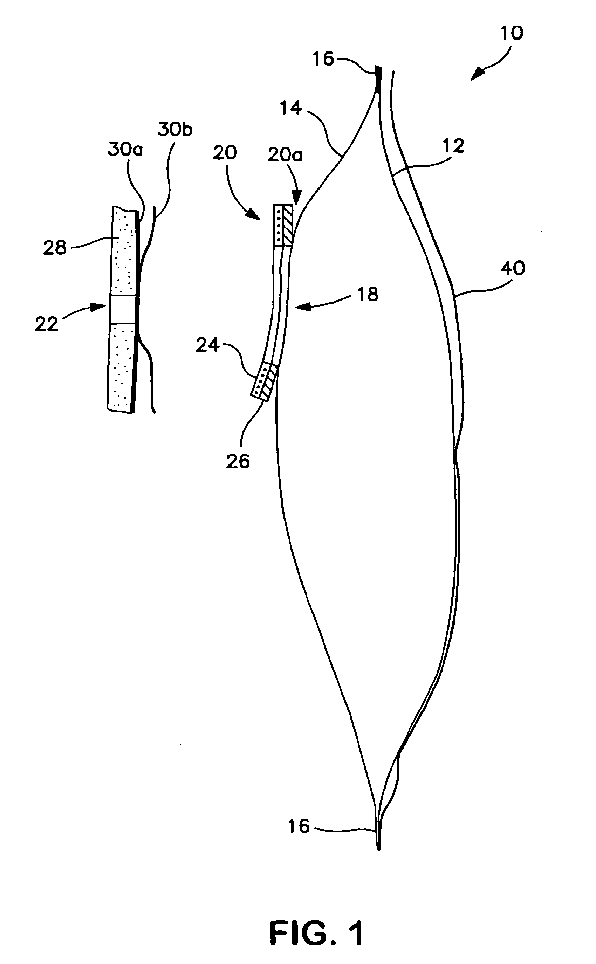

Figs. 1-5 may illustrate a first embodiment of the invention in the form of a medical pouch 10. The pouch 10 may be a collection pouch, for example, an ostomy pouch.

The pouch 10 may generally comprise a front wall 12 and a rear wall 14 joined by a plastics weld 16. The front wall 12 and/or the rear wall 14 may comprise a material that is biodegradable and/or water soluble and/or water dispersible. Suitable materials include, by way of example only, polycaprolactone, polylactic acid (PLA), aliphatic polyester (e.g., Estar Bio made by Eastman, Ecoflex made by BASF, Biomax made by du Pont), copolymers of 3-hydroxybutyrate (e.g., PHBV made by Monsanto), and poly(hydroxyalkonates) homopolymers and copolymers (e.g., Nodax made by Proctor and Gamble), starch based polymers (e.g., Materbi made by Novamont), poly(vinyl alcohol) based polymers, alkaline soluble polymers (e.g., acrylic acid copolymers made by Belland), etc.

The front and/or rear walls 12 and 14 may further comprise a barrier material to obstruct transpiration of odours through the pouch wall. A suitable barrier material is polyvinylidene chloride (PVDC) or poly(vinyl alcohol) or polyacrylonitrile. The barrier material may be coated on the pouch wall material, for example by spraying, or it may be included as a layer of a laminar film.

The front and/or rear walls 12 and 14 may further comprise a comfort layer (not shown). The comfort layer may be carried directly on the respective wall 12 or 14, or the comfort layer may comprise a cover sheet that is secured to the wall 12 or 14, for example, at the peripheral weld 16. The comfort layer may comprise a material that is biodegradable and/or water soluble and/or water dispersible. The comfort layer may comprise a non-woven material, e.g. cotton, viscose, poly(vinyl alcohol), poly(lactic acid), poly(caprolactone), polylactic acid (PLA), aliphatic polyester (e.g., Estar Bio made by Eastman, Ecoflex made by BASF, Biomax made by du Pont), copolymers of 3-hydroxybutyrate (e.g., PHBV made by Monsanto), and poly(hydroxyalkonates) homopolymers and copolymers (e.g., Nodax made by Proctor and Gamble), starch based polymers (e.g., Materbi made by Novamont), poly(vinyl alcohol) based polymers, alkaline soluble polymers (e.g., acrylic acid copolymers made by Belland), etc.

The rear wall 14 may contain an entrance aperture 18 through which collected matter, such as body waste, enters the pouch 10. A collar 20 may be secured to the rear wall 14 around the entrance aperture 18. A purpose of the collar 20 may be to provide a separable fastening to a body attachment wafer 22 (described later). The collar 20 may be secured to the rear wall 14 by any suitable, generally permanent means, for example, by welding or by a strong adhesive. The entire collar 20 may be secured to the rear wall 14, or a peripheral portion 20a of the collar 20 may be unsecured (as indicated in Fig. 1). The unsecured portion 20a may allow for easier manipulation by hand and/or may allow for a greater degree of freedom of movement of the pouch 10 while still supporting the weight of the pouch 10. The collar 20 may be at least partly flexible and/or resiliently flexible. The collar 20 may include a projecting portion in the form of a finger grip 25, by which the collar may be gripped.

The collar 20 may comprise an adhesive layer 24 carried on a substrate 26. The thickness of the adhesive layer 24 may be about 0.3 mm (about 12 mils) or less, for example, about 0.2 mm (about 8 mils) or less, or about 0.1 mm (about 4 mils) or less. The thickness of the adhesive layer 24 may be at least about 0.0025 mm (about 0.1 mils), for example, at least about 0.025 mm (about 1 mil). A preferred thickness range may be from about 0.025 mm (about 1 mil) to about 0.1 mm (about 4 mils).

The adhesive layer 24 may comprise a pressure sensitive adhesive. Suitable pressure sensitive adhesives include, by way of example only, acrylate based adhesives, poly(ethylene vinyl acetate) (EVA) based adhesives, polyolefin based adhesives, styrenic block copolymers based adhesives, silicone based adhesives, water dispersive adhesives (e.g., AQ polymers and/or Sulfopolyester made by Eastman), polyurethane adhesives, etc.

The thickness of the substrate 26 may be about 0.5 mm (about 20 mils) or less, for example, about 0.4 mm (about 16 mils) or less, or about 0.3 mm (about 12 mils) or less, or about 0.25mm (about 10 mils). The substrate may be of a material that is biodegradable and/or water soluble and/or water dispersible. Suitable materials may include, for example, polyvinylalcohol (PVOH), polycaprolactone, polylactic acid, aliphatic polyester, copolymers of 3-hydroxybutyrate, starch based polymers, poly(hydroxyalkonates), etc.

Additionally or alternatively, the combined thickness of the adhesive layer 24 and the substrate 26 may be about 0.8 mm (about 32 mils) or less, for example, about 0.6 mm (about 24 mils) or less, or about 0.4 mm (about 16 mils) or less.

It is to be noted that the thicknesses of the adhesive layer 24 and the substrate 26, are exaggerated in Fig. 1, to enable these layers to be seen distinctly in the schematic drawing.

With the above construction, at least about 50%, or even greater, for example, about 75%, of the pouch material may be biodegradable and/or water soluble and/or water dispersible.

The body attachment wafer 22 may generally comprise a layer of a medical grade skin adhesive 28 and one or more plastics backing layers 30. The adhesive 28 may comprise one or more hydrocolloid polymers. The backing layers 30 may cover the rear adhesive surface of the adhesive 28, and/or may provide a landing surface (or "landing zone") for adhesive engagement by the collar 20. If a single backing layer 30 is used to cover the rear of the adhesive 28 and to provide a landing surface, the collar 20 may be adhered to the wafer 22 in a plane defined generally by the rear of the layer of adhesive 28. If two (or more) backing layers 30a and 30b are used (as in Fig. 1), then the outer backing layer 30b may provide a landing "platform" that may be free to move and/or float and/or flex independently of the shape of the layer of adhesive 28. Such a design may enable the degree of coupling between the pouch 10 and the wafer 22 to be reduced, while still supporting the weight of the pouch. Reducing the degree of coupling may be advantageous in avoiding any tendency of the pouch weight to peel the adhesive 28 away from the wearer's skin. Reducing the degree of coupling may also provide increased comfort for the wearer, and increased isolation of the separation forces applied to the sensitive stoma area if the pouch 10 is removed from the wafer 22 while the wafer is worn on the body.

The body attachment wafer 22 may be similar to that described in EP-A-1033952 and/or EP-A-0793951.

The pouch 10 and the body attachment wafer 22 may be supplied ready connected, as a one-piece item. When the wearer desires to dispose of the pouch 10, the pouch 10 and the connected wafer 22 may be peeled from the skin. Thereafter, the wafer 22 may be separated by peeling the collar 20 away from the wafer 22. The pouch 10 may then be disposed of easily by flushing in a water closet. The relatively thin collar 20 may enable the pouch 10 to be flushed away easily. The material of the collar 20 may soften in water and/or at least partly dissolve, which further facilitates flushing. Yet the collar can provide for a secure and reliable fastening between the pouch 10 and the wafer 22 during use of the pouch 10. Once the pouch 10 has been flushed away, the wafer 22 (which may be relatively clean) may be disposed of separately, for example, in a rubbish bin.

In a further form as a one-piece item, the wafer 22 may be omitted, and the pouch 10 attached to the body by the adhesive layer 24 of the collar 20.

As an alternative to a one-piece item, the pouch 10 and the body attachment wafer 22 may be supplied as separate units, as a two-piece item. The adhesive 24 on the collar 20 may permit repeated adhesion and separation between the pouch 10 and the wafer 22, to enable the pouch 10 to be repositioned on the wafer 22, or interchanged with a replacement pouch 10. When the wearer desires to dispose of the pouch 10, the pouch 10 may be peeled by its collar 20 from the wafer 22 while the wafer 22 remains worn on the body. The pouch 10 may then be disposed of easily in a water closet, as discussed above. If desired, a replacement pouch 10 may be adhered to the wafer 22.

The pouch 10 includes a tear strip or tear tab 40 by which a portion 42 of the pouch 10 may be torn open to further facilitate flushing. The portion 42 may be hatch or flap portion of the pouch wall. The tear tab 40 may be attached to either the front wall 12 or the rear wall 14 of the pouch. In this embodiment, it may be preferred to attach the tear tab 40 to the front wall 12, because the aperture 18 is already provided in the rear wall 14.

The tear tab 40 is secured to the pouch wall 14 by one or more regions or lines of attachment 44, for example by welding or by a strong adhesive. When the tear tab 40 is pulled away from the wall, tears are created in the wall material along at least some of the lines of attachment 44, releasing the portion 42. The lines of attachment include a tear initiation line 44a, and first and second diverging propagation lines 44b and 44c. One or both of the first and second propagation lines 44b and 44c may intersect the tear initiation line 44a at an obtuse angle. The lines of attachment may further include other propagation lines 44d and 44e. At least a portion of the tear initiation line 44a may be relatively thin, for example, between about 1 and about 5 mm in width. The tear initiation line 44a may enable a single tear to be initiated relatively easily. The other tear propagation lines 44b-e may be relatively thin, or they may be thicker. Tearing may occur outside the tear propagation lines 44b-e. The propagation lines 44b-e may enable the single initial tear to propagate outwardly in different directions, around the portion 42 to be opened. The tear propagation lines 44b-e definea closed loop shape. For example, the closed loop shape may be generally quadrilateral (e.g., diamond, or rectangle) or other general polygon, circle or oval shape. In the present embodiment, the tear lines 44 may define a hollow shape, the central region of which may be unattached to the pouch wall. However, in other embodiments using a closed loop shape, substantially the entire area of the closed loop shape may be attached to the pouch wall if desired. Tearing may occur around the periphery of the closed loop shape.

Positioning of the portion 42 to be torn open near or at a lower portion of the pouch may be especially useful for rapid and/efficient discharge of the pouch contents, for example, into WC water. The pouch contents may normally collect by falling to the bottom of the pouch. Tearing open the pouch in this region may allow the pouch contents to exude rapidly under the internal weight and/or internal pressure of the pouch contents.

The tear strip panel 40 may use a thicker film, as compared to the pouch wall material to which it is joined together by, for example, welding. The tear strip panel 40 may also use a different material. A stronger material may be used so that the tearing force will open up the pouch upon pulling the tear strip instead of breaking the tear strip panel itself.

The force required to tear open the pouch 10 using the tear tab 40 may be at least about 0.79 N/cm (2 N/in) and/or not more than about 7.87 N/cm (20 N/in) using a tearing speed of 2540 mm/min based on ASTM F88. This value may apply to one, some, or all, of the tears opened by the tear strip. Such a range has been found to provide adequate pouch strength and security during use of the pouch 10, yet sufficiently easy tearing when the pouch 10 is to be disposed of.

The tear tab 40 may be longer (e.g., significantly longer) than the extent of the region or lines of attachment 44. For example, the tear tab 40 may extend over at least a majority of the height of the pouch 10. The tear initiation line 44a may start below the entrance aperture 18 and/or at or below about halfway down the height of the pouch 10. Use of a relatively long tear tab 40 can increase the leverage or peeling or tearing effect of tab 40. It can also mean that the wearer may hold the tab at a position remote from the portion 42 that is torn open, which may be advantageous for hygiene reasons. A portion 45 of the tear tab 40 may be spaced from or remote from the portion 42 configured to be torn open. The portion 45 may be an upper part of the tear tab 40. Additionally or alternatively, the portion 45 may be a grip portion by which the user may grip the tear tab 40 in use to tear the pouch open. The portion 45 may be secured temporarily in a stowed condition by one or more breakable connections 46. The breakable connection(s) 46 may attach the portion 45 to the pouch envelope, e.g. in the peripheral weld seam 16, at one or more points 46. Each breakable connection may be formed by a partial or spot weld, or by a relatively weak adhesive. The breakable connection may also be formed by a frangible part of the tab 40 itself. The portion 45 of the tear tab may be deployed without tearing the pouch wall, for example, by pulling on an extension tab 47 of the portion 45 of the tear tab 40 to release the portion 45.

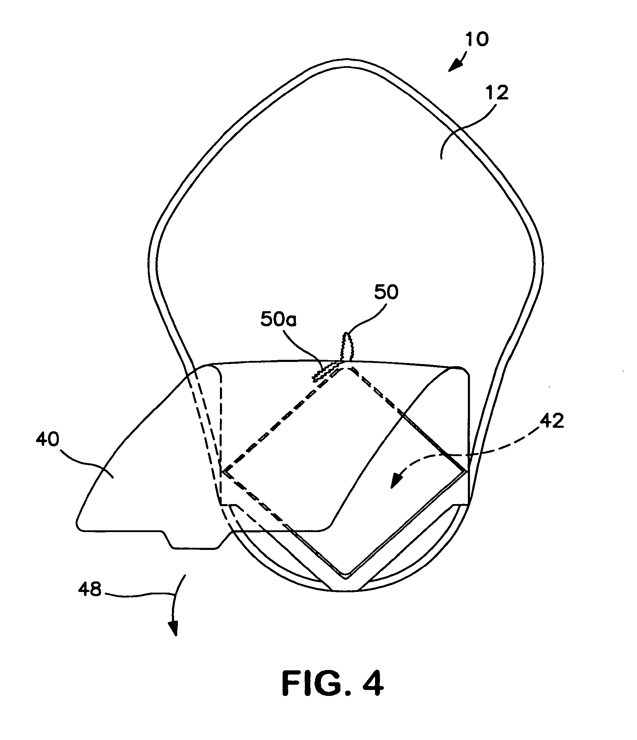

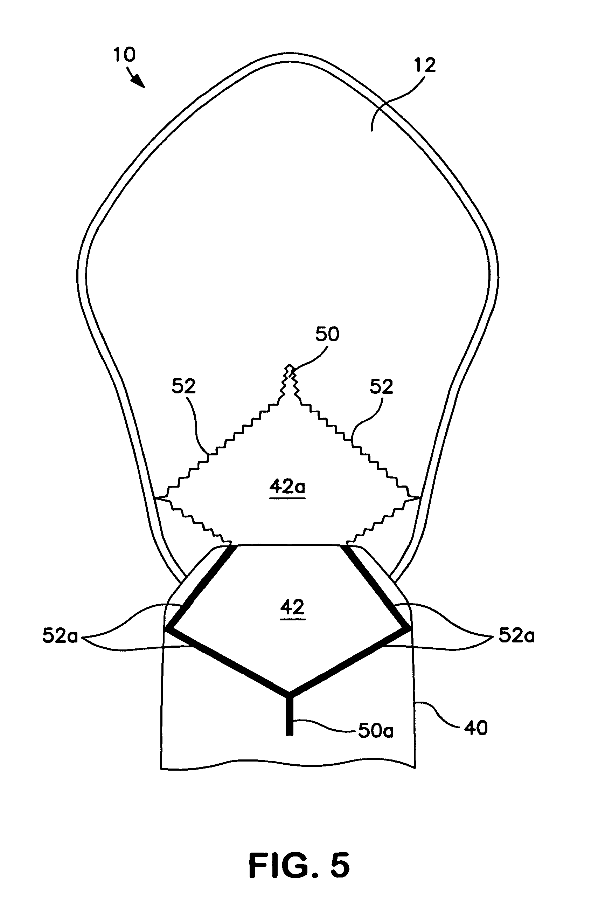

Figs. 4 and 5 illustrate deployment of the tear tab 40 in the direction of the arrow 48. Referring to Fig. 4, an initial tear 50 may be created corresponding to the tear initiation line 44a. The torn out portion 50a of the pouch wall may remain attached to the tear tab 40. Referring to Fig. 5, further pulling of the tear tab 40 may create diverging tears 52 propagating along the tear propagation lines 44b and 44c (and optionally 44d and 44e) at least partly around the portion 42. The torn out portion 42, and the torn out lines of attachment 52a may remain attached to the tear tab 40, creating a relatively large aperture 42a in the pouch wall 12. The tear tab 40 may remain attached to the lower portion of the pouch 10 (as in Fig. 5) or the tear tab 40 may be completely torn away.

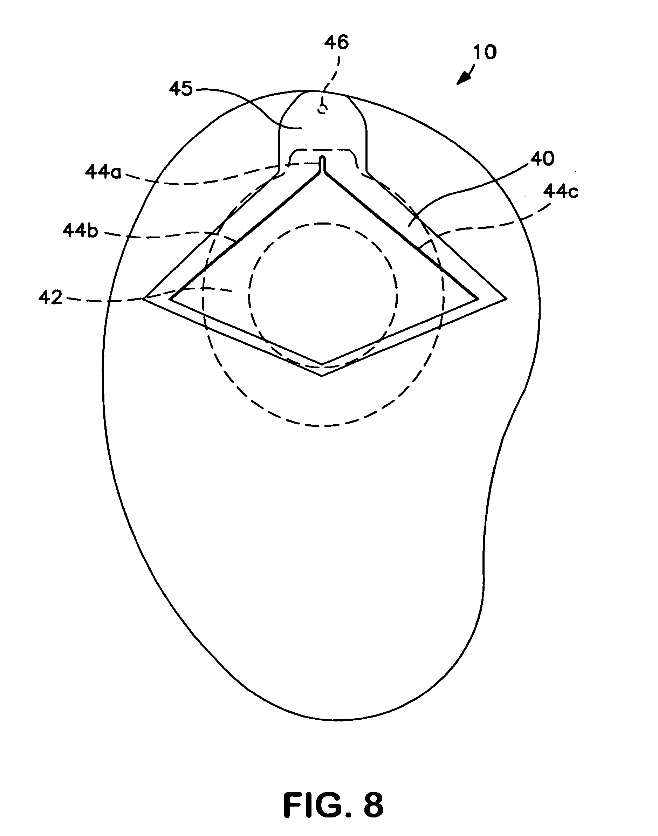

There are other patterns of tear strip that can be designed to allow for easy and/or non-splashing pouch opening, and further examples may be illustrated in Figs. 8, 9, and 10. Like reference numerals are used where appropriate. Where not described, the examples of Figs. 8-10 may include inherently any of the features of the foregoing embodiments as desired.

Referring to Fig. 8, the portion 42 configured to be torn open may be positioned in an upper part of the pouch. The portion 42 may thus be remote from the part of the pouch containing the collected faecal matter. Such positioning may avoid faecal matter splashing or falling out immediately when the portion 42 is torn open, which might be desirable for certain types of ostomy pouch applications. Yet the large portion 42 may allow efficient venting of air from the pouch to reduce its buoyancy for disposal in a WC. The large portion 42 may also allow the contents of the pouch to be emptied easily when desired by the ostomate, or in the turbulence of WC flushing. The tear tab 40 may have a generally quadrilateral shape, with a grip portion extension 45. The grip portion 45 may optionally be secured temporarily by a breakable connection 46. The shape of the portion 42 may be similar to that described above.

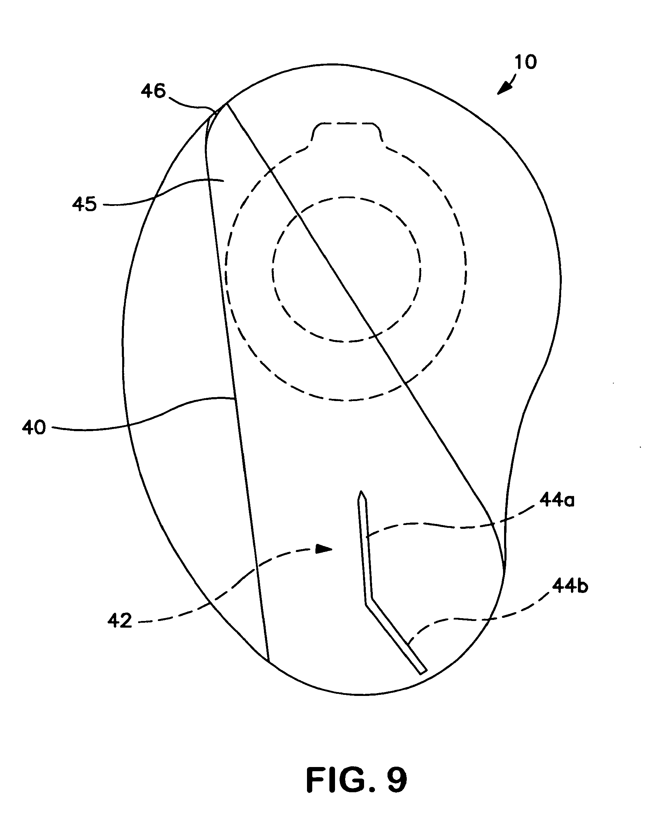

Referring to Fig. 9, which shows an arrangement outside the scope of the invention, the portion 42 may consist of the tear initiation line 44a and a tear propagation line 44b intersecting the tear initiation line 44a at an obtuse angle. Such a shape may be referred to as a vertical "hockey-stick" shape. The portion 42 may be located towards the lower end of the pouch. The portion 42 may itself be relatively narrow in width so as to reduce the risk of contact with faecal matter when the pouch is torn open. The portion 42 may consist of more than a single straight line, to promote venting of gas and/or faecal matter over a relatively wide area, despite the portion 42 itself being relatively narrow.

The tear tab 40 may have a tear-drop-like shape, and may extend substantially the entire height of the pouch. The tear tab may be secured by a breakable connection 46 in a similar manner to the first embodiment.

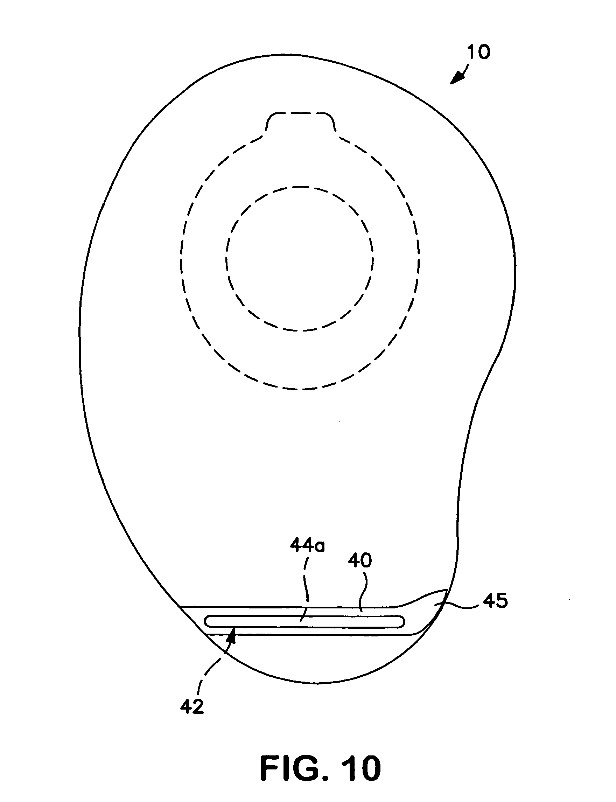

Referring to Fig. 10, which also shows an arrangement outside the scope of the invention, the portion 42 may consist substantially of a single tear line 44a. The portion 42 may be located near the lower end of the pouch. The portion 42 may be relatively narrow in width so as to reduce the risk of contact with faecal matter when the pouch is torn open. The tear tab 40 may comprise a grip portion 45 that is above the level of the portion 42 configured to be torn open, at least when the pouch is in a normal upright condition. Such location of the grip portion 45 may also reduce the risk of contact with faecal matter. The configuration of the grip portion 45 may also fit within the profile of the pouch, while allowing a relatively wide portion 42 relative to the width of the pouch. The portion 42 may extend over at least the majority of the dimension of the pouch in the region of the tear tab 40. As in the arrangements illustrated in Figs. 1-7 and 9, positioning the portion 42 near or at the lower end of the pouch 10 may allow efficient discharge of faecal matter from the pouch.

For other minor implementation details of the tear tab 40, reference may be made to US 5976118.

In use of any of the foregoing pouches, prior to placing the pouch 10 in a water closet for disposal, the user may suspend the pouch 10 over the water closet, and may tear open the portion 42, as described above. This may allow at least some of the pouch contents to empty directly into the toilet water. It may also reduce the chances of air becoming trapped in the pouch during flushing, which might otherwise hinder flushing away of the pouch 10. Once the pouch 10 has been torn open, the pouch 10 may be dropped into the water closet, and flushed away.

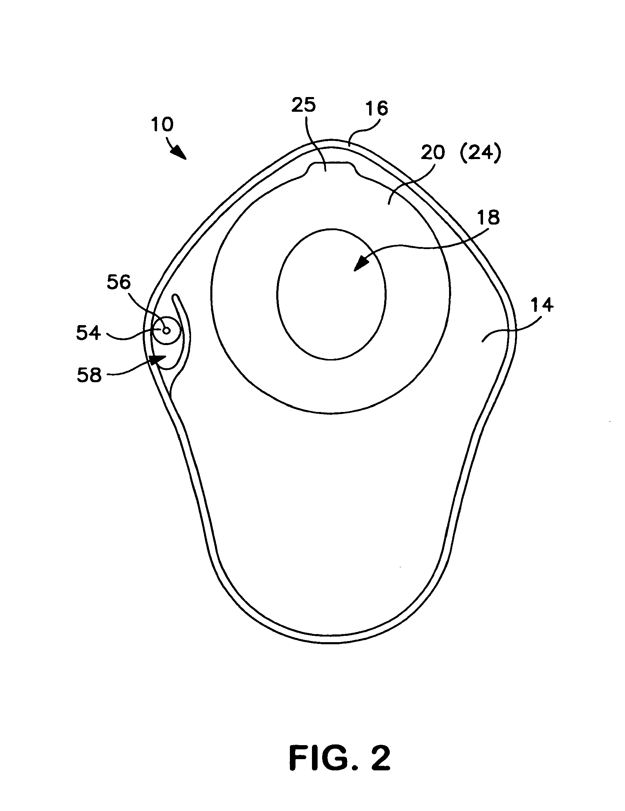

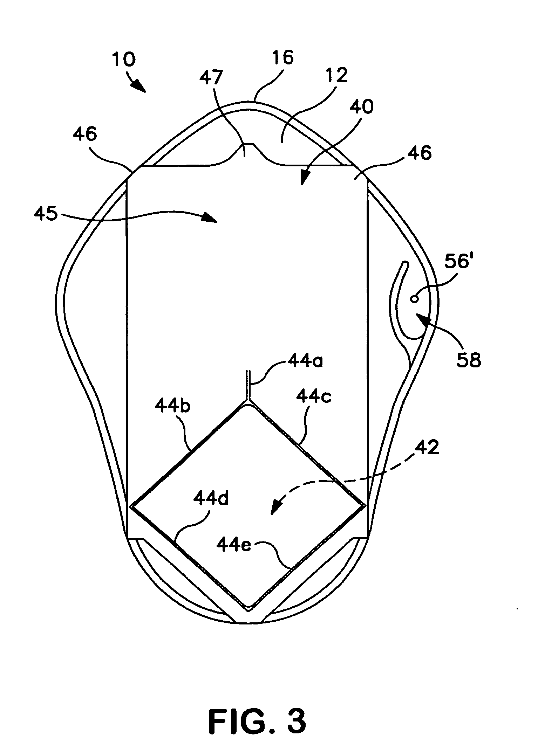

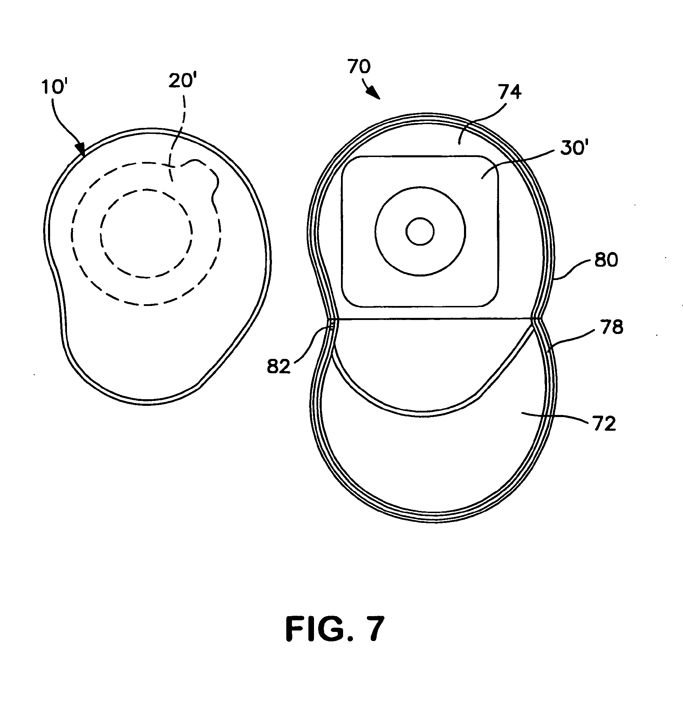

The pouch 10 may further comprise a deodorising filter 54 communicating with a flatus vent 56. The filter 54 may be secured to the exterior of the pouch 10, for example, on the front wall 14. Prior to disposal in the water closet, the wearer may optionally peel off the filter 54. Alternatively, the filter 54 may be secured within a non-removable filter compartment (depicted at 58 in Fig. 2 only), and may be sufficiently small to be flushable as part of the pouch 10. Fig. 3 illustrates the compartment 58 without any filter, which may be more suitable for an unfiltered vent 56. The pouch 10 may have an asymmetric shape, for example a kidney shape (as illustrated in Fig. 7) .

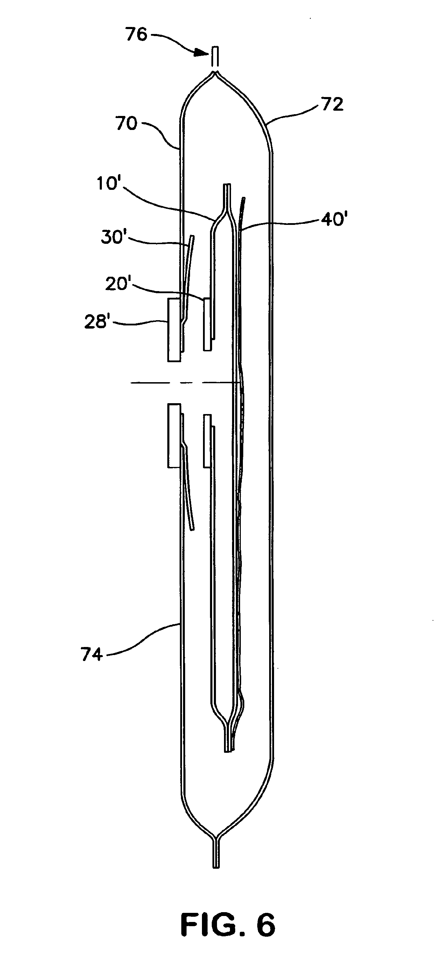

Figs. 6 and 7 illustrate a second embodiment in the form of a twin-pouch (or "pouch in a pouch") implementation. The second embodiment may include an inner pouch 10' and an outer pouch 70. The second embodiment may include many of the features of the first embodiment, and corresponding reference numerals are used where appropriate. The inner pouch 10' may be similar to, or the same as, the pouch 10 of the first embodiment. The outer pouch 70 may include many of the features of the body attachment wafer 22 of the first embodiment, in particular, the skin adhesive 28' and the backing layer(s) 30' providing a landing zone for the collar 20' of the inner pouch 10'.

The pouches 10' and 70 may have an asymmetric shape, for example a kidney shape (as illustrated in Fig. 7). Alternatively, the pouches 10' and 70 may have a generally symmetric shape, for example, similar to the shape illustrated in Figs. 2-5.

The inner pouch 10' may be provided as a replaceable inner that may be disposed of easily by flushing in a water closet, and replaced by a new inner pouch 10'. The enhanced flushability of the inner pouch 10' enables the inner pouch to be disposed of very easily. The outer pouch 70' may be provided as a more substantial cover to protect the inner pouch 10' during use. The outer pouch 70' may be intended to be used multiple times. The outer pouch 70 may comprise a front wall 72 and a rear wall 74. The outer pouch 70 may be openable, to facilitate insertion and removal of the inner pouch 10'. For example, a fastener 76 (Fig. 6) may be provided in a seam between the front wall 72 and the rear wall 74. The fastener may have any suitable form, for example, a sliding zipper fastener. Referring to Fig. 7, the fastener may include complementary zipper tracks 78 and 80 provided on the front and rear walls 72 and 74, respectively, and a zipper slider 82.

Other constructional features of the outer pouch 70 may be found in US 10/630,575.

Another method to facilitate the flushing of a pouch in a toilet is by the use of a carrier sleeve or disposal bag. Pouches, with or without the tear strip design, can be placed in a carrier sleeve or bag prior to flushing. The sleeve or bag may be configured to form a slippery layer when exposed to water, thereby sliding on surfaces that might otherwise cause clogging of the flow passage in a toilet. The material of such a carrier sleeve or disposal bag may include polyvinyl alcohol or highly absorbent tissue paper.

Other constructional features of the carrier sleeve or disposal bag may be found in US Patent No. 4,830,187

It will be appreciated that although the tear tab 40 and the thin collar 20 have been used in combination, other pouches in accordance with the present invention may implement just one of these features in isolation.

It will also be appreciated that the invention may provide significant advantages in enabling pouches to be disposed of conveniently by flushing in a water closet.

The foregoing description is merely illustrative of preferred forms of the invention. Many modifications may be made within the scope of the invention, as defined in the appended claims.

## Claims

1. An ostomy pouch (10) comprising a pouch envelope (12, 14), and a tear strip (40) for tearing open a portion of the pouch envelope, the tear strip being joined to the envelope in one or more regions of attachment (44a-e), said regions of attachment comprising a tear initiation line (44a) and first and second diverging propagation lines (44b, 44c) diverging from the tear initiation line (44a), wherein the first and second propagation lines (44b, 44c) define a closed loop shape.

2. The pouch according to claim 1, wherein one or both of the first and second propagation lines (44b, 44c) intersect the tear initiation line (44a) at an obtuse angle.

3. The pouch according to claim 1 or claim 2, wherein the tear strip (40) is attached to the pouch by one or more welds.

4. The pouch according to any preceding claim, wherein the closed loop shape is generally quadrilateral.

5. The pouch according to any preceding claim, wherein the tear strip comprises a deployable portion (45) attached in a stowed condition by a breakable connection (46), the breakable connection being configured to enable the deployable portion to be deployed without tearing the pouch envelope, by breaking the breakable connection.

6. The pouch according to claim 5, wherein in the stowed condition, the deployable portion (45) is attached by the breakable connection (46) to the pouch envelope.

7. The pouch according to claim 5 or 6, wherein the breakable connection (46) is selected from: a weld; and a weak adhesive.

8. The pouch according to any of claims 5 to 7, wherein the breakable connection (46) is spaced from a region (42) of the pouch wall configured to be torn open.

9. The pouch according to any of claims 5 to 8, wherein the tear strip (40) extends from generally adjacent to one extremity of the pouch to generally adjacent to another extremity of the pouch in the region where the tear strip (40) is located.

10. The pouch according to any preceding claim, configured such that a force required to tear open the pouch along at least one tear line, by using the tear strip, is between about 0.79 N/cm (2 N/in) and about 7.87 N/cm (20 N/in) when measured at a tearing speed of 2540 mm/min.

11. The pouch according to claim 10, wherein the force required to tear open the pouch along each tear line opened by pulling the tear strip, is between about 0.79 N/cm (2 N/in) and about 7.87 N/cm (20 N/in) when measured at a tearing speed of 2540 mm/min.

12. The pouch according to any preceding claim, wherein the pouch envelope is configured to be disposable by flushing in a water closet.

13. The pouch according to claim 12, further comprising a carrier sleeve or disposal bag into which the pouch envelope may be placed, the carrier sleeve or disposal bag comprising material that becomes slippery upon immersion in water to facilitate the pouch flushing in a toilet.

14. The pouch according to any preceding claim wherein the pouch envelope comprises at least 50% material that is one or more of biodegradable, water disintegrateable, and water dispersible.

15. The pouch according to any preceding claim, wherein the pouch envelope has an entrance aperture, and the pouch further comprises an adhesive bearing collar located at the entrance aperture, the collar having a thickness of about 0.8 mm (about 32 mils) or less.

16. The pouch according to any preceding claim, wherein the pouch envelope has an entrance aperture, and the pouch further comprises a collar located at the entrance aperture, the collar carrying an adhesive layer having a thickness of about 0.4 mm (about 16 mils) or less.

17. The pouch according to claim 15 or 16, wherein the thickness of the adhesive layer is between about 0.013 mm (about 0.5 mil) and about 0.2 mm (about 8 mils).

18. The pouch according to any preceding claim, wherein the pouch envelope has an entrance aperture, and the pouch further comprises a collar located at the entrance aperture, the collar comprising a substrate carrying an adhesive layer, the substrate having a thickness of about 0.5 mm (about 20 mils) or less.

19. The pouch according to claim 18 wherein the thickness of the substrate is about 0.4 mm (about 16 mils).

20. The pouch according to any of claims 15 to 19, further comprising a body attachment wafer fastened or fastenable to the collar by the adhesive on the collar.

21. The pouch according to claim 20, wherein the pouch is a one-piece pouch, and the body attachment wafer is separable from the pouch by breaking the adhesive engagement between the collar and the body attachment wafer.

22. The pouch according to claim 20, wherein the pouch is a two-piece pouch, and the body attachment wafer is releasably fastenable to the collar by the adhesive on the collar.

23. The pouch according to any preceding claim, wherein the pouch further comprises a second pouch envelope for removably receiving the first mentioned pouch envelope.

## Patentansprüche

1. Stomabeutel (10) mit einer Beutelhülle (12, 14) und einem Aufreißstreifen (40) zum Aufreißen eines Abschnitts der Beutelhülle, wobei der Aufreißstreifen mit der Hülle in einem oder mehreren Befestigungsbereichen (44a-e) verbunden ist, die Befestigungsbereiche eine Anreißlinie (44a) sowie eine erste und eine zweite divergierende Weiterreißlinie (44b, 44c) aufweisen, die von der Anreißlinie (44a) divergieren, wobei die erste und zweite Weiterreißlinie (44b, 44c) eine geschlossene Schleifenform festlegen.

2. Beutel nach Anspruch 1, wobei die erste und/oder zweite Weiterreißlinie (44b, 44c) die Anreißlinie (44a) in einem stumpfen Winkel schneiden.

3. Beutel nach Anspruch 1 oder Anspruch 2, wobei der Aufreißstreifen (40) durch eine oder mehrere Schweißstellen am Beutel befestigt ist.

4. Beutel nach einem der vorstehenden Ansprüche, wobei die geschlossene Schleifenform allgemein vierseitig ist.

5. Beutel nach einem der vorstehenden Ansprüche, wobei der Aufreißstreifen einen entfaltbaren Abschnitt (45) aufweist, der in einem verstauten Zustand durch eine zerbrechliche Verbindung (46) befestigt ist, wobei die zerbrechliche Verbindung so konfiguriert ist, dass sie ermöglicht, den entfaltbaren Abschnitt ohne Aufreißen der Beutelhülle zu entfalten, indem die zerbrechliche Verbindung zerbrochen wird.

6. Beutel nach Anspruch 5, wobei im verstauten Zustand der entfaltbare Abschnitt (45) durch die zerbrechliche Verbindung (46) an der Beutelhülle befestigt ist.

7. Beutel nach Anspruch 5 oder 6, wobei die zerbrechliche Verbindung (46) aus einer Schweißstelle und einem schwachen Kleber ausgewählt ist.

8. Beutel nach einem der Ansprüche 5 bis 7, wobei die zerbrechliche Verbindung (46) von einem Bereich (42) der Beutelwand beabstandet ist, der so konfiguriert ist, dass er aufgerissen wird.

9. Beutel nach einem der Ansprüche 5 bis 8, wobei sich der Aufreißstreifen (40) aus der allgemeinen Nähe zu einer Extremität des Beutels zur allgemeinen Nähe zu einer weiteren Extremität des Beutels in dem Bereich erstreckt, in dem der Aufreißstreifen (40) liegt.

10. Beutel nach einem der vorstehenden Ansprüche, der so konfiguriert ist, dass eine Kraft, die zum Aufreißen des Beutels an mindestens einer Aufreißlinie mit Hilfe des Aufreißstreifens erforderlich ist, zwischen etwa 0,79 N/cm (2 N/Inch) und etwa 7,87 N/cm (20 N/Inch) in der Messung mit einer Aufreißgeschwindigkeit von 2540 mm/min liegt.

11. Beutel nach Anspruch 10, wobei die Kraft, die zum Aufreißen des Beutels an jeder Aufreißlinie erforderlich ist, die durch Ziehen am Aufreißstreifen geöffnet wird, zwischen etwa 0,79 N/cm (2 N/Inch) und etwa 7,87 N/cm (20 N/Inch) in der Messung mit einer Aufreißgeschwindigkeit von 2540 mm/min liegt.

12. Beutel nach einem der vorstehenden Ansprüche, wobei die Beutelhülle so konfiguriert ist, dass sie durch Wegspülen in einem Wasserklosett entsorgbar ist.

13. Beutel nach Anspruch 12, ferner mit einer Traghülse oder Entsorgungstüte, in der die Beutelhülle platziert werden kann, wobei die Traghülse oder Entsorgungstüte Material aufweist, das bei Eintauchen in Wasser rutschig wird, um das Wegspülen des Beutels in einer Toilette zu erleichtern.

14. Beutel nach einem der vorstehenden Ansprüche, wobei die Beutelhülle mindestens 50 % Material aufweist, das biologisch abbaubar, wasserzersetzbar und/oder wasserdispergierbar ist.

15. Beutel nach einem der vorstehenden Ansprüche, wobei die Beutelhülle eine Eintrittsöffnung hat und der Beutel ferner eine an der Eintrittsöffnung liegende, Kleber tragende Manschette aufweist, wobei die Manschette eine Dicke von höchstens etwa 0,8 mm (etwa 32 Milli-Inch) hat.

16. Beutel nach einem der vorstehenden Ansprüche, wobei die Beutelhülle eine Eintrittsöffnung hat und der Beutel ferner eine an der Eintrittsöffnung liegende Manschette aufweist, wobei die Manschette eine Klebeschicht mit einer Dicke von höchstens etwa 0,4 mm (etwa 16 Milli-Inch) trägt.

17. Beutel nach Anspruch 15 oder 16, wobei die Dicke der Klebeschicht zwischen etwa 0,013 mm (etwa 0,5 Milli-Inch) und etwa 0,2 mm (etwa 8 Milli-Inch) liegt.

18. Beutel nach einem der vorstehenden Ansprüche, wobei die Beutelhülle eine Eintrittsöffnung hat und der Beutel ferner eine an der Eintrittsöffnung liegende Manschette aufweist, wobei die Manschette ein Substrat aufweist, das eine Klebeschicht trägt, und das Substrat eine Dicke von höchstens etwa 0,5 mm (etwa 20 Milli-Inch) hat.

19. Beutel nach Anspruch 18, wobei die Dicke des Substrats etwa 0,4 mm (etwa 16 Milli-Inch) beträgt.

20. Beutel nach einem der Ansprüche 15 bis 19, ferner mit einer Körperbefestigungsscheibe, die durch den Kleber auf der Manschette an der Manschette befestigt oder befestigbar ist.

21. Beutel nach Anspruch 20, wobei der Beutel ein einteiliger Beutel ist und die Körperbefestigungsscheibe vom Beutel trennbar ist, indem der Klebeeingriff zwischen der Manschette und der Körperbefestigungsscheibe aufgelöst wird.

22. Beutel nach Anspruch 20, wobei der Beutel ein zweiteiliger Beutel ist und die Körperbefestigungsscheibe durch den Kleber auf der Manschette an der Manschette lösbar befestigbar ist.

23. Beutel nach einem der vorstehenden Ansprüche, wobei der Beutel ferner eine zweite Beutelhülle zum entfernbaren Aufnehmen der erstgenannten Beutelhülle aufweist.

## Revendications

1. Poche de stomie (10) comprenant une enveloppe de poche (12, 14) et une bande déchirable (40) pour déchirer une portion de l'enveloppe de poche, la bande déchirable étant reliée à l'enveloppe dans une ou plusieurs régions de fixation (44a-e), lesdites régions de fixation comprenant une ligne d'initiation de déchirure (44a) et des première et deuxième lignes de propagation divergentes (44b, 44c) divergeant à partir de la ligne d'initiation de déchirure (44a), où les première et deuxième lignes de propagation (44b, 44c) définissent une forme de boucle fermée.

2. Poche selon la revendication 1, dans laquelle une ou les deux des première et deuxième lignes de propagation (44b, 44c) se croisent avec la ligne d'initiation de déchirure (44a) à un angle obtus.

3. Poche selon la revendication 1 ou la revendication 2, dans laquelle la bande déchirable (40) est fixée à la poche par une ou plusieurs soudures.

4. Poche selon l'une quelconque des revendications précédentes, dans laquelle la forme de boucle fermée est généralement quadrilatérale.

5. Poche selon l'une quelconque des revendications précédentes, dans laquelle la bande déchirable comprend une portion déployable (45) attachée dans un état rentré par une connection rupturable (46), la connection rupturable étant configurée pour permettre le déploiement de la portion déployable sans déchirer l'enveloppe de la poche, en rompant la connection rupturable.

6. Poche selon la revendication 5, dans laquelle à l'état rentré, la portion déployable (45) est fixée par la connection rupturable (46) à l'enveloppe de poche.

7. Poche selon la revendication 5 ou 6, dans laquelle la connection rupturable (46) est sélectionnée parmi: une soudure; et un adhésif faible.

8. Poche selon l'une quelconque des revendications 5 à 7, dans laquelle la connection rupturable (46) est espacée d'une région (42) de la paroi de poche configurée pour être déchirée.

9. Poche selon l'une quelconque des revendications 5 à 8, dans laquelle la bande déchirable (40) s'étend depuis un emplacement généralement adjacent à une extrémité de la poche à un emplacement généralement adjacent à une autre extrémité de la poche dans la région où la bande déchirable (40) est située.

10. Poche selon l'une quelconque des revendications précédentes, configurée de façon qu'une force requise pour déchirer la poche le long d'au moins une ligne de déchirure, en utilisant la bande déchirable, est entre environ 0,79 N/cm (2 N/in) et environ 7,87 N/cm (20 N/in) lorsqu'elle est mesurée à une vitesse de déchirure de 2540 mm/min.

11. Poche selon la revendication 10, dans laquelle la force requise pour déchirer la poche le long de chaque ligne de déchirure ouverte en tirant sur la bande déchirable, est entre environ 0,79 N/cm (2 N/in) et environ 7,87 N/cm (20 N/in) lorsqu'elle est mesurée à une vitesse de déchirure de 2540 mm/min.

12. Poche selon l'une quelconque des revendications précédentes, dans laquelle l'enveloppe de poche est configurée pour être mise au rebut par rinçage dans des toilettes.

13. Poche selon la revendication 12, comprenant en outre un manchon de support ou un sachet dans lequel l'enveloppe de poche peut être placée, le manchon de support ou le sachet de mise au rebut comprenant un matériau qui devient glissant lors de l'immersion dans de l'eau pour faciliter l'évacuation de la poche par la chasse d'eau des toilettes.

14. Poche selon l'une quelconque des revendications précédentes, dans laquelle l'enveloppe de la poche comprend au moins 50% de matériau qui est un ou plusieurs parmi biodégradable, apte à s'intégrer dans l'eau et dispersible dans l'eau.

15. Poche selon l'une quelconque des revendications précédentes, dans laquelle l'enveloppe de poche possède une ouverture d'entrée, et la poche comprend en outre un collier porteur d'adhésif situé à l'ouverture d'entrée, le collier ayant une épaisseur d'environ 0,8 mm (environ 32 mils) ou moins.

16. Poche selon l'une quelconque des revendications précédentes, dans laquelle l'enveloppe de poche possède une ouverture d'entrée, et la poche comprend en outre un collier situé à l'ouverture d'entrée, le collier supportant une couche adhésive d'une épaisseur d'environ 0,4 mm (environ 16 mils) ou moins.

17. Poche selon la revendication 15 ou 16, dans laquelle l'épaisseur de la couche adhésive est entre environ 0,013 mm (environ 0,5 mil) et environ 0,2 mm (environ 8 mils).

18. Poche selon l'une quelconque des revendications précédentes, dans laquelle l'enveloppe de poche possède une ouverture d'entrée, et la poche comprend en outre un collier situé à l'ouverture d'entrée, le collier comprenant un substrat supportant une couche adhésive, le substrat ayant une épaisseur d'environ 0,5 mm (environ 20 mils) ou moins.

19. Poche selon la revendication 18, dans laquelle l'épaisseur du substrat est d'environ 0,4 mm (environ 16 mils).

20. Poche selon l'une quelconque des revendications 15 à 19, comprenant en outre une plaquette de fixation de corps fixée ou pouvant être fixée au collier par l'adhésif sur le collier.

21. Poche selon la revendication 20, dans laquelle la poche est une poche en une pièce, et la plaquette de fixation de corps peut être séparée de la poche en rompant l'engagement adhésif entre le collier et la plaquette de fixation de corps.

22. Poche selon la revendication 20, dans laquelle la poche est une poche en deux pièces, et la plaquette de fixation de corps peut être fixée amoviblement au collier par l'adhésif sur le collier.

23. Poche selon l'une quelconque des revendications précédentes, dans laquelle la poche comprend en outre une deuxième enveloppe de poche pour recevoir amoviblement l'enveloppe de poche mentionnée en premier.
